# EUROPEAN PATENT APPLICATION

(11) **EP 4 375 313 A1**
(43) Date of publication of application: **29.05.2024**
(21) Application number: 22892620.0
(22) Date of filing: 28.10.2022
(51) Int. Cl.: C08J 3/12, A61F 2/16, A61L 15/24, A61L 27/16, A61L 27/34, A61L 29/04, A61L 31/04, C08F 20/56, C08J 5/18, C08L 33/26, G02C 7/04

(54) **POLYDIMETHYLACRYLAMIDE POWDER, POLYDIMETHYLACRYLAMIDE SOLUTION, AND METHOD FOR PRODUCING POLYDIMETHYLACRYLAMIDE POWDER**

(30) Priority: 09.11.2021 JP 2021182341; 31.05.2022 JP 2022088240
(71) Applicant: Toray Industries, Inc., Tokyo 103-8666 (JP)
(72) Inventor: KITAGAWA, Rumiko, Otsu-shi, Shiga 520-8558 (JP); NAKAMURA, Masataka, Otsu-shi, Shiga 520-8558 (JP)
(74) Representative: Prüfer & Partner mbB Patentanwälte · Rechtsanwälte
(86) International application number: PCT/JP2022/040356
(87) International publication number: WO 2023/085121

(57) **Abstract**

An object of the present invention is to provide a polydimethylacrylamide powder, a polydimethylacrylamide solution, and a method for producing a polydimethylacrylamide powder, which have extremely good solubility in various solvents and other production raw materials, and can exhibit remarkable hydrophilicity, lubricity, and transparency when used as a raw material of a medical device. The present invention provides a polydimethylacrylamide powder containing polydimethylacrylamide as a main component and having a bulk density of 0.60 g/mL or less, and a method for producing the polydimethylacrylamide powder.

## Description

### TECHNICAL FIELD

The present invention relates to a polydimethylacrylamide powder, a polydimethylacrylamide solution, and a method for producing a polydimethylacrylamide powder.

### BACKGROUND ART

Medical devices using polydimethylacrylamide as a production raw material are extremely excellent in biocompatibility, hydrophilicity, and the like including lubricity of the surface thereof, and are attracting attention in recent years.

In order to enhance the biocompatibility and hydrophilicity of a medical device obtained by molding polydimethylacrylamide as the production raw material, or to enhance transparency of the medical device, it is desired that polydimethylacrylamide has high purity and high compatibility with various solvents and other production raw materials in a production process.

As a means for increasing the compatibility between a polymer such as polydimethylacrylamide and various solvents or other production raw materials, that is, for increasing solubility of the polymer itself, for example, it is conceivable to adjust properties such as a moisture content of the polymer. As an example, in the example of polyvinylpyrrolidone, a method of sufficiently drying a polyvinylpyrrolidone aqueous solution or the like having a high viscosity with a drum dryer or increasing its apparent density is disclosed (Patent Documents 1 to 3).

### PRIOR ART DOCUMENT

### PATENT DOCUMENTS

Patent Document 1: Japanese Patent Laid-open Publication No. 2004-285267
Patent Document 2: Japanese Patent Laid-open Publication No. 2008-095048
Patent Document 3: Japanese Patent Laid-open Publication No. 2009-249473

### SUMMARY OF THE INVENTION

### PROBLEMS TO BE SOLVED BY THE INVENTION

However, a specific means for remarkably increasing the solubility of polydimethylacrylamide has not been known at all so far.

Thus, an object of the present invention is to provide a polydimethylacrylamide powder, a polydimethylacrylamide solution, and a method for producing a polydimethylacrylamide powder, which have extremely good solubility in various solvents and other production raw materials, and can exhibit high hydrophilicity, lubricity, and transparency when used as a raw material of a medical device.

### SOLUTIONS TO THE PROBLEMS

In order to achieve the above object, the present invention provides a polydimethylacrylamide powder containing polydimethylacrylamide as a main component and having a bulk density of 0.60 g/mL or less, and a method for producing the polydimethylacrylamide powder.

### EFFECTS OF THE INVENTION

According to the polydimethylacrylamide powder of the present invention, it is possible to provide a medical device having high transparency, lubricity, and hydrophilicity.

### EMBODIMENTS OF THE INVENTION

A polydimethylacrylamide powder of the present invention contains polydimethylacrylamide as a main component, and has a bulk density of 0.60 g/mL or less.

In the present specification, polydimethylacrylamide refers to a homopolymer of an N,N-dimethylacrylamide monomer or a copolymer of an N,N-dimethylacrylamide monomer and another monomer. Here, when polydimethylacrylamide is a copolymer of an N,N-dimethylacrylamide monomer and another monomer, only the copolymer containing a structural unit derived from the N,N-dimethylacrylamide monomer at a content of 50 mol% or more is referred to as "polydimethylacrylamide". That is, the polydimethylacrylamide in the present specification refers to a polymer in which the content of a structural unit derived from an N,N-dimethylacrylamide monomer is 50 to 100 mol%.

In polydimethylacrylamide, in order to develop moderate viscosity when a polydimethylacrylamide powder is dissolved in a solvent or the like to enhance hydrophilicity and lubricity of a medical device to be obtained, the content of the structural unit derived from an N,N-dimethylacrylamide monomer is preferably 75 to 100 mol%, more preferably 90 to 100 mol%, and still more preferably 100 mol% (homopolymer of N,N-dimethylacrylamide monomer).

Here, "containing as a main component" means that a mass ratio of polydimethylacrylamide in the polydimethylacrylamide powder is 50% or more.

In the present specification, the powder refers to an aggregate of polymer particles in which a plurality of polymer particles form a gap while being in contact with each other. The polydimethylacrylamide powder refers to an aggregate of polydimethylacrylamide particles in which a plurality of polydimethylacrylamide particles form a gap while being in contact with each other.

The polydimethylacrylamide powder of the present invention is required to have a bulk density of 0.60 g/mL or less. Here, the bulk density refers to a density when a container having a certain volume is filled with a polydimethylacrylamide powder and the internal volume thereof is taken as a volume. Specifically, the bulk density can be determined by calculating the bulk density when 10.0 g of the polydimethylacrylamide powder of the present invention is filled in a 250 mL volumetric flask without any tapping.

The bulk density of the polydimethylacrylamide powder of the present invention is preferably 0.0001 g/mL or more, more preferably 0.0005 g/mL or more, more preferably 0.001 or more, more preferably 0.005 g/mL or more, still more preferably 0.01 g/mL or more, particularly preferably 0.05 g/mL or more in order to enhance compatibility with various solvents and other production raw materials of a medical device. The bulk density is preferably 0.50 g/mL or less, more preferably 0.40 g/mL or less, still more preferably 0.20 g/mL or less, particularly preferably 0.10 g/mL or less.

The polydimethylacrylamide powder of the present invention preferably contains polydimethylacrylamide having a weight average molecular weight of 100,000 or more as a main component. When the weight average molecular weight of polydimethylacrylamide contained as a main component is 100,000 or more, sufficient hydrophilicity and lubricity can be imparted to a medical device obtained using the powder as a raw material. The weight average molecular weight of polydimethylacrylamide contained as a main component is preferably 150,000 or more, more preferably 400,000 or more, and still more preferably 600,000 or more. The weight average molecular weight of polydimethylacrylamide contained as a main component is preferably 1.2 million or less, and more preferably 1 million or less from the viewpoint of production stability. The weight average molecular weight referred to herein is a weight average molecular weight in terms of polyethylene glycol measured by a gel permeation chromatography method (aqueous solvent).

Here, "containing as a main component" means that in the polydimethylacrylamide powder, a mass ratio of polydimethylacrylamide having a weight average molecular weight in the specific range is 50% or more.

Examples of other monomers to be copolymerized with the N,N-dimethylacrylamide monomer include compounds having a structure containing an unsaturated group, such as vinyl amide, vinyl imide, vinyl lactam, hydrophilic (meth)acrylate, (meth)acrylamide or a derivative thereof, hydrophilic styrenic compounds, vinyl ether, vinyl carbonate, vinyl carbamate, or vinyl urea.

Specific examples of the compound include N-vinylpyrrolidone, N-vinyl-2-piperidone, N-vinyl-2-caprolactam, N-vinyl-3-methyl-2-caprolactam, N-vinyl-3-methyl-2-piperidone, N-vinyl-4-methyl-2-piperidone, N-vinyl-4-methyl-2-caprolactam, N-vinyl-3-ethyl-2-pyrrolidone, N-vinyl-4,5-dimethyl-2-pyrrolidone, vinyl imidazole, N,N-diethylacrylamide, acrylamide, N,N-bis(2-hydroxyethyl)acrylamide, acrylonitrile, N-isopropylacrylamide, 2-ethyloxazoline, N-(2-hydroxypropyl) (meth)acrylamide, N-(2-hydroxyethyl) (meth)acrylamide, 2-methacryloyloxyethylphosphorylcholine, 3-(dimethyl(4-vinylbenzyl)ammonio)propane-1-sulfonate (DMVBAPS), 3-((3-acrylamidopropyl)dimethylammonio)propane-1-sulfonate (AMPDAPS), 3-((3-methacrylamidopropyl)dimethylammonio)propane-1-sulfonate (MAMPDAPS), 3-((3-(acryloyloxy)propyl)dimethylammonio)propane-1-sulfonate (APDAPS), 3-((3-methacryloyloxy)propyl)dimethylammonio)propane-1-sulfonate (MAPDAPS), N-vinyl-N-methylacetamide, N-vinylacetamide, N-vinyl-N-methylpropionamide, N-vinyl-N-methyl-2-methylpropionamide, N-vinyl-2 -methylpropionamide, N-vinyl-N, N'-dimethylurea, dimethylaminopropyl (meth)acrylamide, dimethylaminoethyl (meth)acrylamide, dimethylaminopropyl (meth)acrylate, dimethylaminoethyl (meth)acrylate, and methoxypolyethylene glycol (meth)acrylate.

Among them, N-vinylpyrrolidone, N-vinyl-2-piperidone, N-vinyl-2-caprolactam, N-vinyl-3-methyl-2-caprolactam, N-vinyl-3-methyl-2-piperidone, N-vinyl-4-methyl-2-piperidone, N-vinyl-4-methyl-2-caprolactam, N-vinyl-3-ethyl-2-pyrrolidone, N-vinyl-4, 5-dimethyl-2-pyrrolidone, vinyl imidazole, N,N-diethylacrylamide, N-isopropylacrylamide, N-(2-hydroxypropyl) (meth)acrylamide, N-vinyl-N-methylacetamide, N-vinyl-acetamide, N-vinyl-N-methylpropionamide, N-vinyl-N-methyl-2-methylpropionamide, N-vinyl-2-methylpropionamide, dimethylaminopropyl (meth)acrylamide, dimethylaminoethyl (meth)acrylamide, dimethylaminopropyl (meth)acrylate or dimethylaminoethyl (meth)acrylate, and methoxypolyethylene glycol (meth)acrylate are preferable, and N-vinylpyrrolidone, N-vinyl-N-methylacetamide or methoxypolyethylene glycol (meth)acrylate is more preferable because it is easy to copolymerize with the N,N-dimethylacrylamide monomer.

A void ratio of the polydimethylacrylamide powder of the present invention is preferably 1.0% or more, more preferably 4.0% or more, and still more preferably 7.0% or more in order to enhance compatibility with various solvents and other production raw materials of medical devices. From the viewpoint of ease of production and good handleability, the void ratio is preferably 90.0% or less, more preferably 80.0% or less, and still more preferably 70.0%. A method for measuring the void ratio will be described later.

A water content of the polydimethylacrylamide powder of the present invention is preferably 0.001% by mass or more, more preferably 0.01% by mass or more, and still more preferably 0.1% by mass or more from the viewpoint of ease of production. In addition, the water content is preferably 10.0% by mass or less, more preferably 8.0% by mass or less, and still more preferably 5.0% by mass or less in order to enhance compatibility with various solvents and other production raw materials of the medical device or transparency of the medical device to be obtained. A method for measuring the water content will be described later.

The polydimethylacrylamide powder of the present invention may contain an N,N-dimethylacrylamide monomer and/or another monomer remaining without being polymerized as an unpolymerized monomer. The content of such an unpolymerized monomer, particularly the content of the unpolymerized N,N-dimethylacrylamide monomer is preferably 0 ppm or more and 230 ppm or less, more preferably 0 ppm or more and 100 ppm or less, still more preferably 0 ppm or more and 50 ppm or less, and particularly preferably 0 ppm or more and 20 ppm or less, in order to suppress a concern about an increase in impurities due to toxicity or hydrolysis. Here, 0 ppm means that the content of the unpolymerized N,N-dimethylacrylamide monomer is not more than a detection limit. A method for measuring the unpolymerized monomer content will be described later.

The polydimethylacrylamide solution of the present invention is a solution obtained by dissolving the polydimethylacrylamide powder of the present invention or a polydimethylacrylamide sheet described later in a solvent. As the solvent, those described later can be preferably used. Since the polydimethylacrylamide powder of the present invention has high compatibility with a solvent, the polydimethylacrylamide powder can be dissolved without remaining undissolved.

By molding the solution of the present invention, a medical device can be obtained. Specifically, a medical device can be obtained by filling a polydimethylacrylamide solution into a mold having a desired shape and then curing the solution. Although the polydimethylacrylamide solution may contain a part of the polydimethylacrylamide remaining without being dissolved, since the polydimethylacrylamide powder of the present invention has high solubility in a solvent, there is no undissolved residue, and a medical device having high hydrophilicity, lubricity, biocompatibility, and transparency can be obtained.

Examples of the medical device includes an ophthalmic lens, a skin covering material, a wound covering material, a skin protective material, a drug carrier for skin, an infusion tube, a gas transport tube, a drainage tube, a blood circuit, a covering tube, a catheter, a stent, a sheath, a biosensor chip, a heart-lung machine, or an endoscope covering material. Among them, since transparency can be remarkably enhanced in addition to expression of sufficient hydrophilicity and lubricity, it is preferable to be used as a raw material for producing an ophthalmic lens. Here, examples of the ophthalmic lens include contact lenses, intraocular lenses, artificial corneas, corneal inlays, corneal onlays, and eyeglass lenses.

Examples of the solvent include water; alcohol-based solvents such as methanol, ethanol, propanol, 2-propanol, butanol, tert-butanol, and tert-amyl alcohol; aromatic hydrocarbon-based solvents such as benzene, toluene, and xylene; aliphatic hydrocarbon-based solvents such as hexane, heptane, octane, decane, petroleum ether, kerosene, ligroin, and paraffin; ketone-based solvents such as acetone, methyl ethyl ketone, and methyl isobutyl ketone; ester-based solvents such as ethyl acetate, butyl acetate, methyl benzoate, dioctyl phthalate, and ethylene glycol diacetate; ether-based solvents such as diethylether, tetrahydrofuran, and dioxane; and glycol ether-based solvents such as ethylene glycol dialkyl ether, diethylene glycol dialkyl ether, triethylene glycol dialkyl ether, tetraethylene glycol dialkyl ether, polyethylene glycol dialkyl ether, a polyethylene glycol-polypropylene glycol block copolymer, and a polyethylene glycol-polypropylene glycol random copolymer. An alcohol-based solvent or a glycol ether-based solvent that can be easily removed from the obtained medical device by water washing is preferable.

As the solvent, various polymerizable monomers may be used as long as polydimethylacrylamide can be dissolved. When the monomer is polymerized in a polydimethylacrylamide solution containing a monomer as a solvent, polydimethylacrylamide is present inside or on the surface of a homopolymer or copolymer obtained from the monomer.

Examples of the monomer for improving the mechanical properties of the obtained homopolymer or copolymer include aromatic vinyl compounds such as styrene, tertbutylstyrene, and α-methylstyrene, and (meth)acrylic acid alkyl esters such as methyl (meth)acrylate, ethyl (meth)acrylate, n-propyl (meth)acrylate, n-butyl (meth)acrylate, tert-butyl (meth)acrylate, isobutyl (meth)acrylate, n-hexyl (meth)acrylate, n-octyl (meth)acrylate, 2-ethylhexyl (meth)acrylate, n-heptyl (meth)acrylate, n-nonyl (meth)acrylate, n-decyl (meth)acrylate, isodecyl (meth)acrylate, n-lauryl (meth)acrylate, tridecyl (meth)acrylate, n-dodecyl (meth)acrylate, cyclopentyl (meth)acrylate, cyclohexyl (meth)acrylate, and n-stearyl (meth)acrylate.

Examples of the monomer for improving the hydrophilicity of the obtained homopolymer or copolymer include methacrylic acid, acrylic acid, itaconic acid, 2-hydroxyethyl methacrylate, 2-hydroxyethyl acrylate, 2-hydroxypropyl methacrylate, 2-hydroxypropyl acrylate, glycerol methacrylate, polyethylene glycol methacrylate, N,N-dimethylacrylamide, N-methylacrylamide, dimethylaminoethyl methacrylate, methylenebisacrylamide, diacetoneacrylamide, N-vinylpyrrolidone, N-vinylcaprolactam, N-vinylacetamide, and N-vinyl-N-methylacetamide.

Examples of the monomer for improving mechanical properties of the obtained homopolymer or copolymer include ethylene glycol dimethacrylate, diethylene glycol dimethacrylate, triethylene glycol dimethacrylate, polyethylene glycol dimethacrylate, trimethylolpropane trimethacrylate, pentaerythritol tetramethacrylate, bisphenol A dimethacrylate, vinyl methacrylate, acryl methacrylate, and acrylates corresponding to these methacrylates, divinylbenzene, and triallyl isocyanurate.

Examples of the monomer for improving oxygen permeability of the obtained homopolymer or copolymer include known silicone monomers.

A method for producing a polydimethylacrylamide powder of the present invention includes: an application step of applying a polydimethylacrylamide solution to a surface of a holder to obtain a coating film; and a drying step of heating and drying the coating film to obtain dried polydimethylacrylamide.

The polydimethylacrylamide solution applied to the surface of the holder in the application step refers to a solution in which polydimethylacrylamide is dissolved in a solvent. Examples of the polydimethylacrylamide solution include a reaction solution obtained after polymerizing an N,N-dimethylacrylamide monomer in an aqueous solvent, and a solution obtained by dissolving a polydimethylacrylamide powder in a solvent. The polydimethylacrylamide solution may contain a part of polydimethylacrylamide remaining without being dissolved, and is preferably a solution in which the entire amount of polydimethylacrylamide is dissolved because it is easy to handle. The polydimethylacrylamide concentration in the polydimethylacrylamide solution is preferably 3 to 40% by mass, more preferably 5 to 30% by mass, and still more preferably 10 to 20% by mass in order to make the viscosity of the solution suitable and to enhance production efficiency of the polydimethylacrylamide powder. The polydimethylacrylamide solution may contain an organic solvent other than the main solvent or various low molecular weight compounds.

The "holder" in the application step refers to a structure or a molded body having a curved surface and capable of holding a coating film of a polydimethylacrylamide solution on the surface.

As the holder, a cylindrical rotating body capable of uniformly obtaining a coating film and continuously performing treatment is preferable. Examples of a dryer suitably used for heating and drying include a drum dryer, a belt dryer, and a shelf type dryer. The drum dryer is preferable because a polydimethylacrylamide powder that is excellent in thermal efficiency, rapidity, and continuous operability and has a lower bulk density can be obtained. Here, the drum dryer refers to a rotary drum that can be heated from the inside thereof using a heat medium such as water vapor, that is, a dryer including a cylindrical rotating body. The drum dryer serves as both the holder and the dryer, and can apply a polydimethylacrylamide solution to a surface (heating surface) of the drum dryer in the form of a thin film to obtain a coating film (application step), and heat and dry the coating film while rotating the drum dryer to peel off the coating film to obtain dried polydimethylacrylamide (drying step). Examples of the drum dryer include a single drum dryer, a double drum dryer, and a twin drum dryer.

As a method for applying the polydimethylacrylamide solution to the surface of the holder in the application step, a suitable method may be selected according to the shape, properties, and the like of the holder, and when the holder is a drum dryer, examples of the method include a splash feed method, a dip feed method, a lower roll transfer method, an upper roll feed method, a side roll feed method, and a multi-roll feed method.

An average thickness of the coating film of the polydimethylacrylamide solution obtained in the application step is preferably 0.01 to 5.00 mm in order to achieve sufficient drying of the dried polydimethylacrylamide obtained in the subsequent drying step and to facilitate peeling of the dried polydimethylacrylamide.

When the holder in the application step is a drum dryer, the dried polydimethylacrylamide obtained after the drying step can be peeled off from the drum dryer using, for example, a scraper knife. The dried polydimethylacrylamide peeled is preferably in the form of a sheet since the dried polydimethylacrylamide is easily formed into a powder and a polydimethylacrylamide powder having a low bulk density can be obtained. The water content of the dried polydimethylacrylamide is preferably 10.0% by mass or less, more preferably 8.0% by mass or less, still more preferably 5.0% by mass or less.

The temperature of the heating and drying in the drying step can be appropriately set according to the temperature of the polydimethylacrylamide solution and a method for supplying the polydimethylacrylamide solution, and is preferably equal to or higher than the boiling point of the solvent of the polydimethylacrylamide solution, and more preferably a temperature higher by 10°C or higher than the boiling point. For example, when the solvent of the polydimethylacrylamide solution is an aqueous solvent, the temperature is preferably 80 to 180°C, more preferably 100 to 170°C, and still more preferably 120 to 160°C. As the heat medium for heating, a general heat medium can be used, and examples thereof include an organic heat medium oil, hot water, water vapor, silicone oil, and an electric heater. Water vapor with high safety and low production cost is preferable.

Although the drying atmosphere may be the air, since polydimethylacrylamide is highly hygroscopic, the relative humidity is preferably 0 to 60%.

The time from the application of the polydimethylacrylamide solution in the application step to the peeling off of the dried polydimethylacrylamide in the drying step can be appropriately set according to the concentration of the polydimethylacrylamide solution, the method for supplying the solution, a dryer used for heat drying, and the like, and is generally preferably 0.1 to 180 minutes.

The dried polydimethylacrylamide obtained by the drying step is peeled off from the holder to obtain a sheet-like polydimethylacrylamide sheet. The method for peeling the polydimethylacrylamide sheet from the holder is not limited, and the peeling can be performed using, for example, a scraper knife or the like.

Preferred ranges of the content of the structural unit derived from the N,N-dimethylacrylamide monomer, the weight average molecular weight, the void ratio, and the water content in polydimethylacrylamide contained in the polydimethylacrylamide sheet are the same as those in the case of the polydimethylacrylamide powder described above.

A polydimethylacrylamide powder can be obtained through a pulverization step of pulverizing a polydimethylacrylamide sheet using a pulverizer. Since the polydimethylacrylamide sheet obtained by the method of the present invention can obtain a polydimethylacrylamide powder having a proper bulk density in a single pulverization step, the obtained polydimethylacrylamide powder may be used as it is, or the bulk density may be appropriately adjusted using a pulverizer. Here, examples of the pulverizer include a paddle type pulverizer, a rotary blade type pulverizer, a hammer mill, a pin mill, a ball mill, a mortar mill, and a vibration mill.

The method for producing a polydimethylacrylamide powder of the present invention preferably further includes a polymerization step of polymerizing N,N-dimethylacrylamide in an aqueous solvent to obtain a polydimethylacrylamide solution. By providing the polymerization step, the reaction solution as a polydimethylacrylamide solution obtained by the polymerization step is subjected to the application step, whereby operation of redissolving polydimethylacrylamide after polymerization and/or purification in a solvent is omitted, and efficiency of the entire production process can be improved.

Examples of the aqueous solvent in the polymerization step include alcohol-based solvents such as water, methanol, ethanol, propanol, 2-propanol, butanol, tert-butanol, tert-amyl alcohol, 3,7-dimethyl-3-octanol, and tetrahydrolinalool. As the aqueous solvent, water, tert-butanol, tert-amyl alcohol, or 3,7-dimethyl-3-octanol is more preferable, and water is still more preferable, since these aqueous solvents hardly inhibit polymerization and have high safety.

In the polymerization of N,N-dimethylacrylamide in the polymerization step, it is preferable to add a thermal polymerization initiator or a photopolymerization initiator represented by a peroxide or an azo compound in order to promote the initiation of polymerization by reaction with a monomer. As the thermal polymerization initiator, a thermal polymerization initiator that is dissolved in a desired reaction solvent and has optimal decomposition characteristics at a desired reaction temperature may be selected, and generally, an azo-based initiator or a peroxide-based initiator having a 10 hour half-life temperature of 40 to 120°C is preferable. Examples of a photoinitiator include carbonyl compounds, peroxides, azo compounds, sulfur compounds, halogen compounds, and metal salts. These polymerization initiators may be used in combination.

In order to improve the hydrophilicity and lubricity of a medical device to be obtained by causing the polydimethylacrylamide to exhibit moderate viscosity when being dissolved in various solvents or other production raw materials after pulverization, the content of the structural unit derived from the N,N-dimethylacrylamide monomer in the polydimethylacrylamide obtained by the polymerization step is preferably 50 to 100 mol%, more preferably 75 to 100 mol%, still more preferably 90 to 100 mol%, and particularly preferably 100 mol% (homopolymer of N,N-dimethylacrylamide monomer).

### EXAMPLES

Hereinafter, the present invention will be specifically described with reference to Examples, but the present invention is not limited thereto. First, analysis methods and evaluation methods will be described below.

### <Weight average molecular weight>

The weight average molecular weight of the polydimethylacrylamide powder was measured under the following conditions.
Apparatus: Prominence GPC system (manufactured by Shimadzu Corporation)
Pump: LC-20AD
Autosampler: SIL-20AHT
Column oven: CTO-20A
Detector: RID-10A
Column: GMPWXL (manufactured by Tosoh Corporation; internal diameter 7.8 mm × 30 cm, particle size: 13 µm)
Solvent: water/methanol = 1/1 (0.1 N lithium nitrate added) Flow rate: 0.5 mL/min
Measurement time: 30 minutes
Sample concentration: 0.1 to 0.3% by mass
Sample injection volume: 100 µL
Standard sample: Polyethylene oxide standard sample (manufactured by Agilent Technologies, Inc.; 0.1 to 1258 kD)

### <Bulk density>

A volume when 10.0 g of the polydimethylacrylamide powder was filled in a 250 mL volumetric flask without any tapping was read, and the mass (g) was divided by the read volume (mL) to calculate the value. An average value of values of N = 3 was taken as the bulk density (g/mL) of the polydimethylacrylamide powder.

### <Content of unpolymerized monomers

The content of the unpolymerized monomer in the polydimethylacrylamide powder was measured under the following conditions using, as a sample, a solution in which 0.1 g of the polydimethylacrylamide powder was dissolved in 10 mL of distilled water.
Apparatus: Prominence high performance liquid chromatography (manufactured by Shimadzu Corporation)
Pump: LC-20AD
UV detector: SPD-20AV (217 nm)
Column: Eclipse XDB-C18USP (manufactured by Agilent Technologies, Inc.)
Mobile phase: water/acetonitrile
Column temperature: 40°C
Measurement time: 20 minutes
Sample injection volume: 10 µL
Flow rate: 1.5 mL/minute
Standard sample for calibration curve: aqueous monomer solution (2 to 100 ppm).

### <Water content>

The polydimethylacrylamide powder was placed in an aluminum dish, and its mass (Ww) was measured. Thereafter, the powder was vacuum-dried at 105°C for 2 hours using a vacuum dryer, and then the mass (Wd) of the powder was measured. From these masses, the water content of the polymer powder of the polydimethylacrylamide powder was calculated by the following formula (1). The average value of the values of N = 3 was taken as the water content. Water content (% by mass) of polydimethylacrylamide powder =100×(Wv/Wd)/Ww

### <Void ratio>

The polydimethylacrylamide powder was held in a holder, and non-destructive three-dimensional observation was performed by X-ray CT under the following conditions.
Apparatus: 3D high resolution X-ray microscope nano 3DX (manufactured by Rigaku Corporation)
X-ray source: Cu
Tube voltage-tube current: 40 kV -30 mA
Detector: S-CMOS camera (lens: 1080,270)
Resolution: 2.534 µm/pix
Analysis software: Avizo (manufactured by Thermo Fisher Scientific Inc.)

Polydimethylamide particles were extracted from observation data acquired under conditions of an observation range of about 2.5 mm square and a resolution of 2.5 µm/pix, and segmentation of a polydimethylamide portion was performed. Thereafter, a contour was extracted along a form of an outer periphery of the polydimethylamide particles using the Compute ambient occlusion function of the analysis software, and segmentation of void portions of the polydimethylamide particles was performed. The volume of the polydimethylamide portion and the volume of the void portion were calculated from a three-dimensional image after the segmentation, and a ratio of the volume of the void portion when the total of both volumes was taken as 100% was taken as the void ratio of the polydimethylacrylamide powder.

### <Sessile drop contact angle>

A sessile drop contact angle of a surface of a medical device was measured using a contact angle measuring device Drop master DM500 (manufactured by Kyowa Interface Science Co., Ltd.) (droplet method) FAMAS Add-in Software (manufactured by Kyowa Interface Science Co., Ltd.). Specifically, after lightly wiping off moisture on the surface of the hemispherical medical device, the medical device was placed on hemispherical polypropylene having a diameter of 14 mm, and used as a measurement sample. A dropping amount of a phosphate buffer solution to be added dropwise was 20 µL. The average value of the values of N = 3 was taken as the sessile drop contact angle.

### <Friction coefficient>

Under the following conditions, the friction coefficient of the surface of a hemispherical medical device in a state of being wetted with a phosphate buffer solution was measured at N = 5, and the average value of the values of N = 5 was taken as the friction coefficient.
Apparatus: Friction tester KES-SE (manufactured by KATO TECH CO., LTD.)
Friction SENS: H
Measurement SPEED: 2 × 1 mm/sec
Friction load: 44 g

### <Total light transmittance>

Using a sheet-like medical device as a measurement sample, a total light transmittance was measured using an SM color computer (model SM-7-CH; manufactured by Suga Test Instruments Co., Ltd.). After lightly wiping off moisture on the surface of the sheet-like medical device, the thickness was measured using an ABC Digimatic Indicator (model ID-C112; manufactured by Mitutoyo Corporation), and a sample having a thickness of 0.50 to 0.60 mm was used as a measurement sample. An acceptance criterion for transparency was set to a total light transmittance of 80% or more.

### <Phosphate buffer solution>

The compositions of the phosphate buffer solutions used in the following Examples and Comparative Examples and the above measurements are as follows.
KCl 0.20g/L
KH₂PO₄ 0.20g/L
NaCl 8.00g/L
Na₂HPO₄ (anhydrous) 1.5g/L
EDTA (ethylenediaminetetraacetic acid) 0.25 g/L

### (Synthesis Example 1) <Aqueous homopolymer solution of N,N-dimethylacrylamide monomer>

A 500 mL four-necked flask equipped with a stirrer, a thermometer, a cooling tube, and a three-way cock was charged with 292.07 g of distilled water and 34.67 g of N,N-dimethylacrylamide (manufactured by KJ Chemicals Corporation; 0.35 mol), and ultrasonic degassing and nitrogen substitution were performed five times. After the completion of nitrogen substitution in a system, a lower portion of the flask was immersed in an oil bath set at 104°C with stirring. From a time point at which the temperature in the flask exceeded 85°C, 23.7 mg of a polymerization initiator V-50 (manufactured by FUJIFILM Wako Pure Chemical Corporation; 0.0875 mmol) dissolved in 20.00 g of distilled water was added in six portions at intervals of 5 minutes. After adding the total amount of the polymerization initiator, the temperature in the flask was kept at 90°C to 95°C for 2 hours to obtain a homopolymer aqueous solution of an N,N-dimethylacrylamide monomer (polymerization step).

### [Example 1]

5 kg of the homopolymer aqueous solution of the N,N-dimethylacrylamide monomer obtained in Synthesis Example 1 was applied to the surface of a rotary drum of a single drum dryer (manufactured by Katsuragi Industry Co., Ltd.; rotary drum dimension ϕ 800 mm × 500 mm, rotary drum surface area 1.25 m², S-0805 type) in the air and dried (application step and drying step), and then peeled off using a scraper knife to obtain sheet-like polydimethylacrylamide. The surface temperature of the rotary drum was 153°C. The obtained sheet-like polydimethylacrylamide was pulverized using a small pulverizer (manufactured by Sansho Co., Ltd.; Force Mill FM-1) to obtain a polydimethylacrylamide powder. The weight average molecular weight, bulk density, unpolymerized monomer content, water content, and void ratio of the obtained polydimethylacrylamide powder were as shown in Table 1.

### [Examples 2 to 5]

5 kg of a homopolymer aqueous solution (manufactured by Osaka Organic Chemical Industry Ltd.; 10% by mass aqueous solution) of an N,N-dimethylacrylamide monomer was dried and pulverized in the same manner as in Example 1 to obtain a polydimethylacrylamide powder. The weight average molecular weight, bulk density, unpolymerized monomer content, water content, and void ratio of the obtained polydimethylacrylamide powder were as shown in Table 1.

### [Comparative Example 1]

200 g of the homopolymer aqueous solution of the N,N-dimethylacrylamide monomer obtained in Synthesis Example 1 was collected in an aluminum dish, applied in the form of a thin film to obtain a coating film (application step), and dried at 60°C for 8 hours in a vacuum dryer (drying step). The obtained sheet-like polydimethylacrylamide was pulverized using a small pulverizer (manufactured by Sansho Co., Ltd.; Force Mill FM-1) to obtain a polydimethylacrylamide powder. The weight average molecular weight, bulk density, unpolymerized monomer content, water content, and void ratio of the obtained polydimethylacrylamide powder were as shown in Table 1.

### [Comparative Examples 2 to 5]

200 g of the homopolymer aqueous solution (manufactured by Osaka Organic Chemical Industry Ltd.; 10% by mass aqueous solution) of the N,N-dimethylacrylamide monomer was dried and further pulverized in the same manner as in Comparative Example 1 to obtain a polydimethylacrylamide powder. The weight average molecular weight, bulk density, unpolymerized monomer content, water content, and void ratio of the obtained polydimethylacrylamide powder were as shown in Table 1.

**[Table 1]**

| | Type of monomer | Monomer structural unit (mol%) | Weight average molecular weight | Bulk density (g/mL) | Content of unpolymerised monomer (ppm) | Water content (% by mass) | void ratio (%) |
|---|---|---|---|---|---|---|---|
| Example 1 | N,N-Dimethylacrylamide | 100 | 620,000 | 0.10 | 220 | 3.5 | 4.9 |
| Example 2 | N,N-Dimethylacrylamide | 100 | 740,000 | 0.08 | 0 (Not more than detection limit) | 3.1 | 23.8 |
| Example 3 | N,N-Dimethylacrylamide | 100 | 250,000 | 0.09 | 0 (Not more than detection limit) | 3.2 | 8.3 |
| Example 4 | N,N-Dimethylacrylamide | 100 | 500,000 | 0.10 | 0 (Not more than detection limit) | 3.3 | 9.7 |
| Example 5 | N,N-Dimethylacrylamide | 100 | 160,000 | 0.08 | 0 (Not more than detection limit) | 3.6 | 1.5 |
| Comparative Example 1 | N,N-Dimethylacrylamide | 100 | 620,000 | 0.65 | 220 | 3.2 | 1.8 |
| Comparative Example 2 | N,N-Dimethylacrylamide | 100 | 740,000 | 0.64 | 0 (Not more than detection limit) | 3.1 | 1.9 |
| Comparative Example 3 | N,N-Dimethylacrylamide | 100 | 760,000 | 0.61 | 0 (Not more than detection limit) | 3.3 | 3.4 |
| Comparative Example 4 | N,N-Dimethylacrylamide | 100 | 500,000 | 0.65 | 0 (Not more than detection limit) | 3.1 | 2.5 |
| Comparative Example 5 | N,N-Dimethylacrylamide | 100 | 250,000 | 0.68 | 0 (Not more than detection limit) | 3.2 | 3.1 |

### <Preparation of polymerization stock solution containing silicone monomer>

### <Preparation of polymerization stock solution 1>

1.0 part by mass of the polydimethylacrylamide powder obtained in Example 1, 38.0 parts by mass of a silicone monomer represented by the following formula (a), 9.0 parts by mass of 2-ethylhexyl acrylate (manufactured by Tokyo Chemical Industry Co., Ltd.), 0.1 parts by mass of dimethylaminoethyl acrylate (manufactured by Kohjin Co., Ltd.), 49.9 parts by mass of 2-hydroxyethyl methacrylate (manufactured by Tokyo Chemical Industry Co., Ltd.), 2.0 parts by mass of triethylene glycol dimethacrylate (manufactured by Tokyo Chemical Industry Co., Ltd.), and 5,000 ppm of a photoinitiator "IRGACURE" (registered trademark) 819 (manufactured by Nagase & Co., Ltd.) with respect to the total mass of these monomers were prepared. All the polydimethylacrylamide powder among the above-mentioned raw materials was added to 40.0 parts by mass of t-amyl alcohol, and then stirred at 200 rpm for 2.5 hours in an incubator at 60°C. Thereafter, after all the other raw materials were added, the mixture was stirred at 200 rpm for 2.0 hours in an incubator at 60°C, and then it was visually confirmed that there was no undissolved polydimethylacrylamide powder. Thereafter, the obtained mixture was filtered using a PP syringe filter (manufactured by Membrane Solutions) having a pore size of 0.45 µm and a diameter of 13 mm to obtain a polymerization stock solution 1.

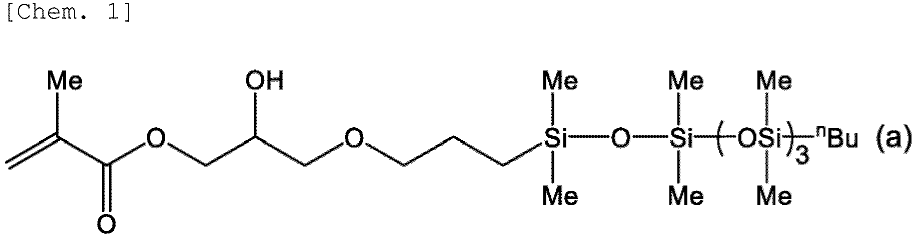

### Preparation of polymerization stock solutions 2, 3, 6, 7, 9, 10 and 11>

Polymerization stock solutions 2, 3, 6, 7, 9 and 10 were respectively obtained by performing the same operations as those for the preparation of the polymerization stock solution 1 except that the polydimethylacrylamide powder obtained in Example 1 was replaced with those obtained in Examples 2 and 3 or Comparative Examples 1, 2, 4 and 5. The results of visually confirming the undissolved polydimethylacrylamide in each preparation process are as shown in Table 2.

A polymerization stock solution 11 was obtained by performing the same operation as in the preparation of the polymerization stock solution 1 except that the polydimethylacrylamide powder was not added (0.0 parts by mass) and the amount of 2-hydroxyethyl methacrylate was 50.9 parts by mass.

### <Preparation of polymerization stock solution containing no silicone monomer>

### <Preparation of polymerization stock solution 4>

99.0 parts by mass of 2-hydroxyethyl methacrylate (manufactured by Tokyo Chemical Industry Co., Ltd.), 1.0 part by mass of the polydimethylacrylamide powder obtained in Example 4, and 2,500 ppm of a photoinitiator "IRGACURE" 819 with respect to the total mass of these were provided, and all of these were mixed and stirred at 200 rpm for 1.0 hour in an incubator at 60°C. Thereafter, it was visually confirmed that there was no undissolved polydimethylacrylamide powder. The obtained mixture was filtered using a PP syringe filter (manufactured by Membrane Solutions) having a pore size of 0.45 µm and a diameter of 13 mm to obtain a polymerization stock solution 4.

### <Preparation of polymerization stock solutions 5, 8, and 12>

Polymerization stock solutions 5 and 8 were obtained by performing the same operations as those for the preparation of the polymerization stock solution 4 except that the polydimethylacrylamide powder obtained in Example 4 was replaced with that obtained in Example 5 or Comparative Example 3. The results of visually confirming the undissolved polydimethylacrylamide powder in each preparation process are as shown in Table 2.

A polymerization stock solution 12 was obtained by performing the same operation as in the preparation of the polymerization stock solution 4 except that the polydimethylacrylamide powder was not added (0.0 parts by mass) and the amount of 2-hydroxyethyl methacrylate (manufactured by Tokyo Chemical Industry Co., Ltd.) was 100 parts by mass.

### <Molding of sheet-like medical devices

Between two glass plates having a size of 4 cm square and a thickness of 3.0 mm, a silicone sheet having a thickness of 0.5 mm hollowed out at the central portion in a size of 2.5 cm square was sandwiched as a spacer to prepare a sample. In a glove box under a nitrogen atmosphere, the central portion of the silicone sheet was filled with the polymerization stock solution 1, and the polymerization stock solution 1 was cured by light irradiation for 20 minutes using a fluorescent lamp (manufactured by TOSHIBA Lighting & Technology Corporation; FL6D, 8.4 kilolux).

Since the polymerization stock solution 1 contained a silicone monomer, a cured product was immersed in a 60% by mass isopropanol (IPA) aqueous solution at 60°C for 30 minutes to be peeled off from the glass plate, and then further immersed in an 80% by mass IPA aqueous solution at 60°C for 2 hours to extract impurities such as a residual monomer. Thereafter, the cured product was further immersed in a 50% by mass IPA aqueous solution, a 25% by mass IPA aqueous solution, and water in this order for 30 minutes each to be hydrated. The cured product after hydration was immersed in a phosphate buffer solution in a 4 mL vial, placed in an autoclave together with the vial, and subjected to boiling treatment at 121°C for 30 minutes to obtain a sheet-like medical device 1.

Sheet-like medical devices 2 to 12 were obtained by performing the same operation as described above except that the polymerization stock solution 1 was replaced with the polymerization stock solutions 2 to 12. When the polymerization stock solution did not contain a silicone monomer, the cured product after light irradiation was immersed in pure water at 90°C for 1 hour instead of the IPA aqueous solution to be peeled off from the glass plate, and further immersed in pure water at 90°C for 2 hours to be boiled.

### <Molding of hemispherical medical device>

Hemispherical medical devices 1 to 12 were obtained by performing the same operation as the molding of the sheet-like medical device except that a recess of a hemispherical mold having a diameter of 14 mm was filled with the polymerization stock solutions 1 to 12.

### <Evaluation of medical device>

The total light transmittance was evaluated for the sheet-like medical devices 1 to 12, and the sessile drop contact angle and the coefficient of friction were evaluated for the hemispherical medical devices 1 to 12. The evaluation results are as shown in Table 2. When each of the polydimethylacrylamide powders obtained in Examples 1 to 5 was used, the total light transmittance as an index of transparency was sufficiently high and a good value was obtained. In addition, it was confirmed that both the sessile drop contact angle as an index of hydrophilicity and the friction coefficient as an index of lubricity were low, and remarkable hydrophilicity and lubricity were exhibited.

**[Table 2]**

| Polymerization stock solution | Silicone monomer | Polydimethyl acrylamide powder used | Undissolved polydimethyl acrylamide powder. | Total light transmittance (%) | Sessile drop contact angle, (°) | Friction coefficient |
|---|---|---|---|---|---|---|
| 1 | Containing | Example 1 | Absent | 81 | 85 | 0.34 |
| 2 | Containing | Example 2 | Absent | as | 77 | 0.35 |
| 3 | Containing | Example 3 | Absent | as | 75 | 0.39 |
| 4 | Free | Example 4 | Absent | 81 | 76 | 0.30 |
| 5 | Free | Example 5 | Absent | 81 | 64 | 0.24 |
| 6 | Containing | Comparative Example 1 | Present | 77 | 93 | 0.53 |
| 7 | Containing | Comparative Example 2 | Present | 80 | 94 | 0.52 |
| 8 | Free | Comparative Example 3 | Present | 77 | 85 | 0.50 |
| 9 | Containing | Comparative Example 4 | Present | 79 | 94 | 0.51 |
| 10 | Containing | Comparative Example 5 | Present | 81 | 94 | 0.53 |
| 11 | Containing | - | - | 89 | 98 | 0.57 |
| 12 | Free | - | - | 85 | 87 | 0.53 |

## Claims

1. A polydimethylacrylamide powder comprising polydimethylacrylamide as a main component,
the polydimethylacrylamide powder having a bulk density of 0.60 g/mL or less.

2. The polydimethylacrylamide powder according to claim 1, wherein the polydimethylacrylamide has a weight average molecular weight of 100,000 or more.

3. The polydimethylacrylamide powder according to claim 1 or 2, wherein the polydimethylacrylamide powder has a void ratio of 1.0% or more.

4. The polydimethylacrylamide powder according to any one of claims 1 to 3, wherein a water content is 10.0% by mass or less.

5. A polydimethylacrylamide sheet comprising polydimethylacrylamide as a main component,
the polydimethylacrylamide sheet having a void ratio of 1.0% or more.

6. A polydimethylacrylamide solution obtained by dissolving the polydimethylacrylamide powder according to any one of claims 1 to 4 or the polydimethylacrylamide sheet according to claim 5 in a solvent.

7. A method for producing a polydimethylacrylamide powder, comprising:
an application step of applying a polydimethylacrylamide solution to a surface of a holder to obtain a coating film; and
a drying step of heating and drying the coating film to obtain dried polydimethylacrylamide.

8. The method for producing a polydimethylacrylamide powder according to claim 7, wherein the holder is a cylindrical rotating body.

9. The method for producing a polydimethylacrylamide powder according to claim 7 or 8, further comprising a polymerization step of polymerizing N,N-dimethylacrylamide in an aqueous solvent to obtain the polydimethylacrylamide solution.

10. The method for producing a polydimethylacrylamide powder according to any one of claims 7 to 9, wherein polydimethylacrylamide contained in the polydimethylacrylamide solution has a weight average molecular weight of 100,000 or more.

11. A method for producing a polydimethylacrylamide sheet, comprising:
an application step of applying a polydimethylacrylamide solution to a surface of a holder to obtain a coating film; and
a drying step of heating and drying the coating film to obtain dried polydimethylacrylamide containing polydimethylacrylamide as a main component.
